# EUROPEAN PATENT APPLICATION

(11) **EP 2 799 864 A1**
(43) Date of publication of application: **05.11.2014**
(21) Application number: 14164914.5
(22) Date of filing: 16.04.2014
(51) Int. Cl.: G01N 33/00, A61K 8/00

(54) **Method to evaluate fragrance compositions**

(30) Priority: 03.05.2013 EP 13166354
(71) Applicant: The Procter & Gamble Company, Cincinnati, OH 45202 (US)
(72) Inventor: Jacob, Timothy John Claud, CARDIFF, CF10 3AX (GB); Douglas, Laura Kathryn, Dumfries, DG1 3GB (GB); Tseng, Hsiu-Er, 105 Taipei City (CN); Bowtell, Philip Andrew, Egham, Surrey TW20 9NW (GB); Holland, Lynette, Anne Makins, Egham, Surrey TW20 9NW (GB)
(74) Representative: Kohol, Sonia

(57) **Abstract**

The present invention relates to a method to evaluate and profile a fragrance composition according to the associated biometric response in humans to the composition. A group of at least 20 test participants is exposed to at least 3 fragrance compositions and at least 4 biometric Autonomatic Nervous System (ANS) parameters are measured. Partition and random/bootstrap forest statistical analysis methods are applied to create a profiling model.

## Description

### FIELD OF THE INVENTION

The invention relates to a method to identify fragrance compositions according to biometric response data.

### BACKGROUND OF THE INVENTION

In addition to fragrance compositions themselves many cosmetic compositions and household cleaning products such as laundry detergents may contain fragrance material(s) in order to provide the composition with a pleasant odour. This can improve the overall consumer acceptance of the composition and or mask unpleasant odours of any of the active components. Alternatively, compositions such as fragrance compositions may be provided for the sole purpose to impart a pleasant odour to the applied substrate such as human skin and which are typically referred to as fine fragrances.

The effect of visual and olfactory stimuli, on the human body, has been reported in the literature.

For example, Collett et al. (J. Autonomic Nervous System 62 (1997), 45-57) reported that the Autonomic Nervous System (ANS), both electrodermal and thermovascular indices are responsive to the application of visual stimuli. Alaoui-Ismaïli et al., (Physiology & Behavior 62 (1997), 713-720) report on the comparison between autonomic response and self reporting of basic emotions response evoked by single raw material odorants. There were no significant differences for two pleasant odourants. Patent no. WO2010/100567 describes the use of eye tracking in order to determine emotional response to olfactory stimuli.

Moreover studies have also shown a degree of correlation between ANS response and self assessment of mood as described by Vernet-Maury et al., (J. Autonomic Nervous System 75(1999); 176-183). The use of decision trees to analyze the data is disclosed however this is based upon numerical methods, not statistical methods. Similarly, Saeki et al. (Int. J. Aromatherapy 15 (2005), 74-80) describe the affect of inhalation of rosemary, lavender and citronella on the autonomic nervous system. Ekman et al., (Science 221 (1983), 1208-1210) describe emotion specific activity generated by constructing facial prototypes of emotion muscle by muscle to distinguish between positive and negative and also among negative emotions using a decision tree model. Elmore, J. (M.Sc. Thesis entitled: "Classifying Emotion Using Streaming of Physiological Correlates of Emotion", pub. Dec. 2012 from the University of Montana) reported the resulting physiological responses to elicited moods. The use of random forest statistical analysis methods to classify the physiological responses is disclosed, however, this is based on the observation of a single subject, not multiple subjects, over an extended period of time (-20 working days).

However, in particular for olfactive stimuli research, the focus of the research has primarily only been directed to differences between both pleasant and unpleasant odours. For example Bensafi et al. (Chem. Senses 27 (2002), 703-709) evaluated the effect of unpleasant (isovaleric acid) and pleasant odours (menthol) on ANS parameters and intensity arousal and pleasantness. A correlation between heart rate and pleasantness and skin conductance and arousal was reported. Bensafi et al. (Neurophysiologie Clinique 32 (2002) 326-332) evaluated the effect of unpleasant (cheese) and pleasant odours (vanilla) on ANS parameters and intensity, arousal, emotion and pleasantness. No relationship was identified between ANS parameters and intensity or pleasantness. In a further study, Bensafi et al. (Neuroscience Letters 319 (2002) 162-166) evaluated the effect of unpleasant (isoamylacetate) and pleasant odours (menthol) on ANS parameters and intensity, arousal, familiarity and pleasantness. Heart rate was observed to be accelerated by unpleasant olfactory stimuli. Sylvain et al. (Emotion 9 (2009), 316-328) evaluated ANS parameters, intensity, hedonics, and familiarity for unpleasant (body odour) and pleasant odours (shampoo) and demonstrated a sequence of events namely that heart rate responses related to novelty and later responses related to pleasantness.

A number of documents have considered perfume raw materials per se. Heuberger et al. (Chem. Senses 26 (2001), 281 - 292) reported the effects of single raw materials limonene and carvone on ANS parameters. Limonene was reported to increase blood pressure and carvone increase blood pressure and pulse rate. Pleasantness ratings were reported as all less than 50 on a 0-100 scale. Dayawansa et al. (Auton. Neurosci. 108 (2003) 79-86) reported that cedrol decreased heart rate and blood pressure. Sayorwan et al. (J. Medical Association of Thailand 95 (2012) 598 - 606) reported the effects of lavender oil inhalation on emotional states, autonomic nervous system, and brain electrical activity; this document reports that lavender oil results in significant decrease in blood pressure, heart rate and skin temperature.

The effect on ANS parameters for natural oils, flower blooms and a number of commercially available fine fragrances has also been reported in the literature.

Saeki et al. (Int. J. Aromatherapy11 (2001) 118-125) describes the testing of lavender, rosemary and citronella (all pleasant natural raw materials) to determine the effect on blood pressure, blood flow, and galvanic skin conductance. Each material was found to cause a different effect on the ANS parameters that could be distinguished from one another.

Jin et al. (Forestry Studies in China 11 (2009) 185-189) describe the ANS parameter recording pre and post exposure to two real flowering blooms (lily and rose). Diastolic pressure was found to decrease with rose bloom and galvanic skin response increase with lily bloom. Other parameters were not affected.

Matsunaga et al. (Neuroendocrinology Letters 32 (2011) 774-780) investigated changes in mood states and autonomic nervous, endocrine and immune activities when autobiographic memories were evoked by a commercially available fragrance identified as nostalgic by the participants. Heart rate decreased, skin conductance level increase, and peripheral interleukin-2 level was decreased (blood sample analysis).

Fragrance, beauty and cosmetic compositions and most household cleaning products are by their nature designed to be olfactory neutral and/or pleasing depending on their specific end use and contain a complex mix of perfume raw materials to impart a pleasant and complex odour to the user or product over a given time period. No conclusive research regarding ANS response to olfactory stimuli has been reported with regard to complex mixtures which are all considered as pleasant.

Thus there is still a need for a non invasive method which is inexpensive and easy to conduct whereby ANS response data can be used to classify and identify pleasant olfactive stimuli containing a complex mixture of perfume raw materials.

It has now been surprisingly found that the application of partition and random/bootstrap forest statistical analysis to ANS biometric data of test participants exposed to a number of fragrance compositions can be used to develop a method of identifying a test fragrance from the associated biometric classification data.

### SUMMARY OF THE INVENTION

The invention is a method to evaluate and profile a fragrance composition according to the associated biometric response in humans to said composition, comprising the steps of:
i) Selecting at least 20 test participants (N), a control and at least 2 fragrance compositions
ii) Exposing each test participant to at least 3 conditions comprising a control and 2 fragrance composition for a predetermined time period;
iii) Measuring at least 4 biometric Autonomic Nervous System (ANS) parameters selected from the group listed below of each test participant before and after the exposure to each of said predetermined fragrance compositions to define an associated biometric fingerprint of each of said predetermined fragrance compositions from said biometric ANS parameters,
iv) Applying partition and random/bootstrap forest statistical analysis methods to said biometric fingerprint data to create a profiling model based upon the data, whereby a predetermined fragrance composition tested by a test participant based upon ANS data can be identified.

### DETAILED DESCRIPTION OF THE INVENTION

The invention relates to a method to create a model based upon biometric response of test participants to olfactory pleasing odours such as fragrance compositions and thereby enable a fragrance to be identified by a biometric fingerprint.

As used herein the term "fragrance" is used to indicate any consumer acceptable pleasantly odouriferous material and/ or composition. Such materials can be readily identified using a consumer test panel (as described hereinafter), wherein at least 15%, preferably at least 20%, more preferably at least 25%, or even more preferably at least 30% consider the material or composition to be excellent or very good or 5 or 4 on a scale of 1 to 5.

Any fragrance material suitable for use in cosmetic compositions may be used herein but the fragrance will most often be liquid at ambient temperatures. Generally, the fragrance material will be present at a level of from about 0.01% to about 40%, by weight, of total composition. Preferably the fragrance material is present at a level of from about 0.1% to about 25%, more preferably from about 0.25% to about 20%, even more preferably from about 0.5% to about 18%, most preferably 1% to 15% by weight, of total composition.

A wide variety of chemicals are known for fragrance uses, including materials such as alcohols, aldehydes, ketones and esters. Commonly, naturally occurring plant oils and exudates comprising complex mixtures of various chemical components are known for use as fragrances.

The fragrance materials useful herein include pro-fragrances such as acetal pro-fragrances, ketal pro-fragrances, ester pro-fragrances, hyrdolyzable inorganic-organic pro-fragrances and mixtures thereof and are readily known to a perfumer. The fragrance material may be released from the pro-fragrances in a number of ways. For example, the fragrance may be released as a result of simple hydrolysis, or by a shift in an equilibrium reaction, or by a pH-change, or by enzymatic release.

The fragrances herein are mixtures of multiple fragrance materials that can be relatively simple in their compositions, comprising a small number of chemicals, or can comprise highly sophisticated complex mixtures of natural and/or synthetic chemical components. Such complex mixtures can be readily produced by perfumers to provide any desired odour. Typically commercially available fragrance composition will contain at least 5, more preferably at least 10, even more preferably at least 15 or more perfume raw materials.

In one embodiment of the present invention, the fragrance composition comprises at least one; preferably at least 5 synthetically derived perfume raw materials. In another embodiment the fragrance composition is substantially free of naturally derived fragrance materials. The use of such naturally derived materials can be readily identified using Carbon 14 dating methods according to ASTM D6866-12.

Preferably the fragrance materials of the present invention will have boiling points (BP) of about 500°C or lower, more preferably about 400°C or lower, even more preferably about 350°C or lower. The BP of many fragrance materials are given in *Perfume and Flavor Chemicals (Aroma Chemicals), Steffen Arctander (1969).* The ClogP value of the fragrance materials useful herein is preferably greater than about 0.1, more preferably greater than about 0.5, even more preferably greater than about 1.0, even more preferably still greater than about 1.2. As used herein the term "ClogP" means the logarithm to the base 10 of the octanol/water partition coefficient. This can be readily calculated from a program called "CLOGP" which is available from Daylight Chemical Information Systems Inc., Irvine CA, USA. Octanol/water partition coefficients are described in more detail in US-A-5,578,563.

Suitable fragrance materials both synthetic and naturally derived are described in detail in Perfume and Flavor Chemicals (Aroma Chemicals) Vol I & II and Perfume and Flavor Materials of Natural Origin by Steffan Arctander, Allured Publishing Corporation 1994. Further suitable fragrance materials can be found in US-A-4,145,184, US-A-4,209,417, US-A-4,515,705, and US-A-4,152,272. Examples of fragrances useful herein include, but are not limited to, plant fragrances such as nutmeg extract, cardamon extract, ginger extract, cinnamon extract, patchouli oil, geranium oil, orange oil, mandarin oil, orange flower extract, cedarwood, vetyver, lavindin, lavender oil, ylang extract, tuberose extract, sandalwood oil, bergamot oil, rosemary oil, spearmint oil, peppermint oil, lemon oil, lavender oil, citronella oil, chamomille oil, clove oil, sage oil, neroli oil, labdanum oil, eucalyptus oil, verbena oil, mimosa extract, narcissus extract, carrot seed extract, jasmine extract, olibanum extract, rose extract and mixtures thereof.

Other examples of suitable fragrance materials include, but are not limited to, chemical substances such as acetophenone, adoxal, aldehyde C-12, aldehyde C-14, aldehyde C-18, allyl caprylate, ambroxan, amyl acetate, dimethylindane derivatives, α-amylcinnamic aldehyde, anethole, anisaldehyde, benzaldehyde, benzyl acetate, benzyl alcohol and ester derivatives, benzyl propionate, benzyl salicylate, borneol, butyl acetate, camphor, carbitol, cinnamaldehyde, cinnamyl acetate, cinnamyl alcohol, cis-3-hexanol and ester derivatives, cis-3-hexenyl methyl carbonate, citral, citronnellol and ester derivatives, cumin aldehyde, cyclamen aldehyde, cyclo galbanate, damascones, decalactone, decanol, estragole, dihydromyrcenol, dimethyl benzyl carbinol, 6,8-dimethyl-2-nonanol, dimethyl benzyl carbinyl butyrate, ethyl acetate, ethyl isobutyrate, ethyl butyrate, ethyl propionate, ethyl caprylate, ethyl cinnamate, ethyl hexanoate, ethyl valerate, ethyl vanillin, eugenol, exaltolide, fenchone, fruity esters such as ethyl 2-methyl butyrate, galaxolide, geraniol and ester derivatives, helional, 2-heptonone, hexenol, α-hexylcinnamic aldehyde, hydroxycitrolnellal, indole, isoamyl acetate, isoeugenol acetate, ionones, isoeugenol, isoamyl iso-valerate, iso E super, limonene, linalool, lilial, linalyl acetate, lyral, majantol, mayol, melonal, menthol, *p*-methylacetophenone, methyl anthranilate, methyl cedrylone, methyl dihydrojasmonate, methyl eugenol, methyl ionone, methyl-β-naphthyl ketone, methylphenylcarbinyl acetate, mugetanol, γ-nonalactone, octanal, phenyl ethyl acetate, phenylacetaldehyde dimethyl acetate, phenoxyethyl isobutyrate, phenyl ethyl alcohol, pinenes, sandalore, santalol, stemone, thymol, terpenes, triplal, triethyl citrate, 3,3,5-trimethylcyclohexanol, γ-undecalactone, undecenal, vanillin, veloutone, verdox and mixtures thereof.

Suitable synthetic fragrance materials include but are not limited to:-Ethyl-1,3,3-trimethyl-2-norbornanol (18368-91-7); 2-Isobutyl-3-methoxypyrazine (24683-00-9); 3-Octen-2-one (1669-44-9); Methyl Abietate (127-25-3); Acetaldehyde propyl phenylethyl acetal (105-57-7); Acetanilide (103-84-4); Iso eugenyl acetate (93-29-8); 2,6,10-Trimethyl-9-undecenal (141-13-9); Allyl isoamyloxyacetate (67634-00-8); Allyl alpha-ionone (79-78-7); 2-tert.Butylcyclohexyloxybutan-2-ol (139504-68-0); 2,5,5-Trimethyl-2-hydroxyoctaline (71832-76-3); n-Amylcyclopentenone (25564-22-1); alpha-Cedrene epoxide (13567-39-0); Phenethyl isoamyl ether (56011-02-0); 1-(3,3-Dimethylcyclohexyl)ethyl-1 formate (25225-08-5); Cyclohexadec-8-en-1-one (88642-03-9); 2-Ethyl-4-(2,2,3-trimethyl-3-cyclopenten-1-yl)-2-buten-1-ol (28219-61-6); Methyl 2-nonenoate (111-79-5); Isoeugenyl benzyl ether (120-11-6); Benzyl laurate (140-25-0); Benzyl n-valerate (10361-39-4); Benzylacetone (2550-26-7); 2,6-Dimethyl-5-heptenal (106-72-9); Formaldehyde cyclododecyl ethyl acetal (58567-11-6); 4,8,8-Trimethyl-4-ethoxy-9-methylenebicyclo[3.3.1]nonane(68845-00-1); 4-(1,1-Dimethylethyl)benzenepropanal (18127-01-0); Cyclohexanone (108-94-1); 1-Methyl-3,4-dioxy(cycloacetonyl)benzene (28940-11-6); 2-Methyl-3-(para-methoxyphenyl)propanal (5462-06-6); 1-Methoxy-2-methyl-5-isopropylbenzene (6379-73-3); 1,1,2,3,3-Pentamethylhexahydro-(5H)-indan-4-one (33704-61-9); Piperonyl acetone (55418-52-5); Cedrenyl acetate (1405-92-1); Trimethylcyclododecadiene epoxide (71735-79-0); Cedryl acetate (isomeric mixtures) (77-54-30); 3-Propylidenephthalide (17369-59-4); 4-Acetyl-6-t-butyl-1,1-dimethylindane (13171-00-1); 12-Oxahexadecanolide (6707-60-4); alpha-Isomethylionone (127-51-5); 2-Heptanone (110-43-0); 2-[(3R,5S,8R)-3,8-dimethyl-1,2,3,4,5,6,7,8-octahydroazulen-5-yl]propan-2-ol (489-86-1); 4-Acetyl-6-t-butyl-1,1-dimethylindane (13171-00-1); Citral diethyl acetal (7492-66-2); Citronellyl nitrile (51566-62-2); Citronellyloxyacetaldehyde (7492-67-3); 3-(4-Methylcycolhex-3-enyl)but-3-enyl acetate (isomers) (6819-19-8); Dodecane nitrile (2437-25-4); 4-tert.Amylcyclohexyl acetate (67874-72-0); Benzofuran (271-89-6); 4-Isopropyl-2-cyclohexenone (500-02-7); Cuminyl nitrile (13816-33-6); Hexahydro-4,7-methanoinden5(6)yl isobutyrate (67634-20-2); Tricyclodecenyl acetate (2500- 83-6); Cyclamen aldehyde (103-95-7); Hexahydro-4,7-methanoinden-5(6)-yl propionate (17511-60-3); Cyclohexyl salicylate (25485-88-5); Decahydro-2-naphthol (825-51-4); trans-4-Decen-1-al (65405-70-1); 2-Pentylcyclopentanone (4819-67-4); Heliotropin (120-57-0); Octahydro-4,7-methanoindan-5(6)-yl acetate (64001-15-6); 3,7-dimethyloct-6-en-3-ol (18479-51); Dihydromyrcenyl acetate (53767-93-4); beta-Ionone (79-77-6); Dihydroroseoxide (13477-62-8); Acetaldehyde dimethyl acetal (534-15-6); Acetaldehyde dimethyl acetal (534-15-6); Dimethyl benzyl carbinyl acetate (151-05-3); 3,7-Dimethyloctyl acetate (20780-49-8); 2,6-dimethylheptan-2-ol (13254-34-7); Dihydromyrcenyl formate (25279-09-8); Diphenylmethane (101-81-5); Dipropylene glycol (25265-71-8); Piperonyl acetone (55418-52-5); Octahydro-4,7-methanoindanilydenebutanal (30168-23-1); 1-(5,5-Dimethyl-1-cyclohexen-1-yl)-4-penten-1-one (56973-85-4); 3-Methyl-5-(2,2,3-trimethyl-3-cyclopenten-1-yl)-4-penten-2-ol (67801-20-1); Linalool ethyl acetal (40910-49-4); Ethyl 3-phenylglycidate (121-39-1); Ethyl maltol (4940-11-8); Ethyl ricinoleate (55066-53-0); Ethyl vanillin (121-32-4); 1,4-Dioxacycloheptadecane-5,17-dione (105-95-3); Eugenyl phenylacetate (10402-33-2); 2-Heptylcyclopentanone (137-03-1); 2,2-Dimethyl-3-(4-ethylphenyl)-propanal (67634-14-4); 3-(3-Isopropyl)phenylbutanal (125109-85-5); 2-(2-Methylpropyl)-4-methyl-tetrahydro-2H-pyran-4-ol (63500-71-0); 2,4,6-Trimethyl-4-phenyl-1,3-dioxane (5182-36-5); Methyl heptine carbonate (111-12-6); Ethyl 2,4-dimethyl-1,3-dioxolane-2-acetate (6290-17-1); 2-sec.Butylcyclohexanone (14765-30-1); Ethyl 2-methyl-1,3-dioxolane-2-acetate (6413-10-1); 2-Methyldecanenitrile (69300-15-8); 4-(2-Furyl)-3-buten-2-one (623-15-4); Hexahydro-4,6,6,7,8,8-hexamethylcyclopenta-gamma-2-benzopyran (1222-05-5); N-Methyl-N-phenyl-2-methyl butyramide (84434-18-4); n-Hexyl ethyl acetoacetate (29214-60-6); 2,6-Dimethylbicyclo[4.4.0]decanol-1 (19700-21-1); Geranyl phenylacetate (102-22-7); trans-Geranylacetone (3796-70-1); Ethyl 2-ethyl-6,6-dimethylcyclo-2-hexene-1-carboxylate (57934-97-1); 2(6)-Methyl-8-(1-methylethyl)bicyclo[2.2.2]oct-5-en-2(3)-yl-1,3-dioxolane (68901-32-6); 5,5,9-Trimethyl-1-ethyltricyclo[8.4.0.0-4,9]-14-oxatetradecane (68611-23-4); 2-Butyl-4,6-dimethyldihydropyran (isomers) (24237-00-1); 2-Methyl-3-(3,4-methylenedioxyphenyl)-propanal (1205-17-0); [2-[1-(3,3-dimethylcyclohexyl)ethoxy]-2-methylpropyl]propanoate (141773-73-1); 3,3-Dimethylcyclohexyl methyl ketone (25304-14-7); 2-Butyl-4,4,6-trimethyl-1,3-dioxane (54546-26-8); Allyl alpha-ionone (79-78-7); 2-Hexenylcyclopentanone (34687-46-2); Hydroxycitronellal dimethyl acetal (141-92-4); Hydroxycitronellol (107-74-4); Hydroxycitronellal (107-75-5); Formaldehyde (indan-1-ol-2-hydroxymethyl) acetal (18096-62-3); 8,8-bis-(2,3-Dihydroindol)-2,6-dimethyl-2-octanol (68908-82-7); Nonenylnitrile (29127-83-1); beta-Ionone (79-77-6); Methyl cyclooctyl carbonate (61699-38-5); Octahydro-2,3,8,8-tetramethyl-2-acetonaphthalene (54464-57-2); (1,7,7-Trimethylbicyclo[2,2,1]hept-2-yl)cyclohexanol (68877-29-2); Vanillyl isobutyrate (20665-85-4); 2-Isobutylquinoline (93-19-6); 3-Acetyl-3,4,10,10-tetramethylbicyclo[4,4,0]decane (54464-57-2); Isocyclocitral (1335-66-6); 1,2,4- (or 1,3,5)-Trimethyl-3-cyclohexene-1-methanol (68527-77-5); 1-(2,4,4-Trimethylcyclohex-1(2)-enyl-1)-2-buten-1-one (39872-57-6); 2-Hexylcyclopent-2-enone-1 (95-41-0); 3-Methyl-2-(2-pentenyl)-2-cyclopenten-1-one (11050-62-7); Cyclohexadecanone (2550-52-9); Methylionones (mixture of isomers) (1335-46-2); Cyclopentanone (120-92-3); 4-Acetoxy-3-pentyl-2H-tetrahydropyran and isomers (18871-14-2); 2-Hexylcyclopentanone (13074-65-2); 3-Butyl-5-methyltetrahydro-2H-pyranyl-4 acetate (38285-49-3); 6-(Z,3-Pentenyl)-tetrahydro-(2H)-pyranone-2 (32764-98-0); 3-Methyl-2-(Z-2-pentenyl)-2-cyclopenten-1-one (488-10-8); 3-Butyl-5-methyltetrahydro-2H-pyranyl-4 acetate (38285-49-3); 2-(2,4-Dimethylcyclohex-3-ene-1-yl)-5-methyl-5-(1-methylpropyl)1,3-dioxane(117933-89-8); 3,3,5,5-Tetramethyl-4-(1-ethoxyvinyl)cyclohexanone (36306-87-3); 3,5,5-Trimethyl-3,8a-ethano-8aH-1-hexahydrobenzopyran-2(3H)-one (21280-29-5); 3,7-Dimethyl-2(3),6-nonadienenitrile (isomers) (61792-11-8); cis-3-Hexenyl methyl carbonate (67633-96-9); 2,2,6-Trimethyl-6-vinyltetrahydropyran (7392-19-0); 3-(4-Methylcyclohex-3-en-1-yl)-butyraldehyde (6784-13-0); Ethyl linalool (10339-55-6); 3,7-Dimethyl-1,6-octadienyl-3 3-phenyl-2-propenoate (78-37-5); Linalyl phenylacetate (7143-69-3); 3-Methyl-5-propyl-2-cyclohexen-1-one (3720-16-9); Methyl cedryl ketone (32388-55-9); 2,6-Dimethyl-2-heptanol (13254-34-7); 3(4)-(4-Hydroxy-4-methylpentyl)cyclohex-3-ene-1-carbaldehyde (31906-04-4); p-tert.Butyl-alpha-methyldihydrocinnamic aldehyde (80-54-6); 7-Formyl-5-isopropyl-2-methylbicyclo[2.2.2]oct-2-ene (67845-30-1); 1-Methylcyclododecyl methyl ether (37514-30-0); 2,4-Dimethyl-5,6-indanyldioxane (27606-09-3); 3-(2-Oxopropyl-1)-2-pentylcyclopentanone (40942-73-2); Methyl 1-methyl-4-isopropylbicyclo[2.2.2]-2-octene-5(6)-carboxylate (68966-86-9); 2,2-Dimethyl-3-(3-methylphenyl)-propanol (103694-68-4); Maltyl butyrate (67860-01-9); Ethyl 2-methylpentanoate (39255-32-8); cis-p-Menthan-7-ol (13828-37-0); 3-Methyl-5-phenyl-1-pentanal (55066-49-4); Dihydroterpinyl acetate (26252-11-9); 6-Methoxy-2,6-dimethylheptanal (62439-41-2); Methyl jasmonate (39924-52-2); Methyl oleate (112-62-9); 1,1-Dimethoxy-2,2,5-trimethyl-4-hexene (67674-46-8); Butyl 2-methylvalerate (6297-41-2); Trimethyl cyclohexanyl methyl ketone (68480-14-8); Methyl 2,6,6-trimethyl-2-cyclohexenylcarboxylate (28043-10-9); alpha-Methylionone (127-42-4); Ethyl 3-methyl-3-phenylglycidate (77-83-8); Propylene glycol (57-55-6); 2,6-Dimethyl-3,5-octadien-2-ol (18479-54-4); (5E)-3-methylcyclopentadec-5-en-1-one (82356-51-2); 3-Methylcyclopentadecanone (541-91-3); Musk ketone (81-14-1); 1,1,3,3,5-Pentamethyl-4,6-dinitroindane (116-66-5); 10-Oxahexadecanolide (1725-01-5); 11-Oxahexadecanolide (3391-83-1); 1-(Prop-2-enoxy) 2-phenylethane (14289-65-7); Isohexenyl cyclohexenyl carboxaldehyde (37677-14-8); 2-Methyl-6-methylene-7-octen-2-ol (543-39-5); 2,2-Dimethyl-3-(3-methylpenta-2,4-dienyl)oxirane (69103-20-4); Diethyl phthalate (84-66-2); 2-[2(4-Methyl-3-cyclohexen-1-yl)propyl]cyclopentanone (95962-14-4); beta-Naphthyl ethyl ether (93-18-5); 1-(2-Methyl-5-isopropyl-2-cyclohexen-1-yl)propanone (31375-17-4); (E)-2,(Z)-6-Nonadienal (557-48-2); Nopol (128-50-7); Nopyl acetate (128-51-8); 6-(2,2,6-Trimethylcyclohexyl)-4-hexanol (70788-30-6); Ocimenyl acetate (72214-23-4); Octanal dimethyl acetal (10022-28-3); Octahydrocoumarin (4430-31-3); Octanal diethyl acetal (54889-48-4); 2,4-Dimethyl-2-(1,1,4,4-tetramethyltetralin-6-yl)-1,3-dioxolane (131812-67-4); 4-tert.Amylcyclohexanone (16587-71-6); 7-methoxy-3,7-dimethyloctan-2-ol (41890-92-0); (2R,4S)-2-methyl-4-propyl-1,3-oxathiane (59323-76-1); p-Hydroxybenzylacetone (5471-51-2); 4-Methylbenzyl acetate (2216-45-7); p-Cresyl isobutyrate (103-93-5); p-Cresyl caprylate (59558-23-5); p-Tolyl phenylacetate (101-94-0); 6-Methyl-tetrahydroquinoline (91-61-2); 2-Nonyn-1-al dimethyl acetal (13257-44-8); 3,7-Dimethyl-1-octanol (106-21-8); 2-Methylphenethyl alcohol (19819-98-8); 2-Cyclohexylidene-2-phenylacetonitrile (10461-98-0); gamma-Undecalactone (104-67-6); Phenylethyl isopropyl ether (68039-47-4); 6-Acetyl-1,1,2,3,3,5-hexamethylindan (15323-35-0); Phenylethyl cyclohexyl ether (80858-47-5); Phenethyl alcohol (60-12-8); 2-Phenoxyethyl isobutyrate (103-60-6); 3-Methyl-5-phenyl-1-pentanol (55066-48-3); 2-Phenoxyethanol (122-99-6); Isoeugenol (97-54-1); 6,6-Dimethylbicyclo[3.1.1]-2-heptenyl-2 propanal (33885-51-7); Phenethyl pivalate (67662-96-8); 4-Methyltricyclo[6.2.1.0]undecan-5-one (41724-19-0); Ethyl 2-(cyclohexyl)propionate (2511-00-4); 3,3-Dimethyl-5-(2,2,3-trimethylcyclopent-3-enyl-1)pent-4-en-2-ol (107898-54-4); (1-Methyl-4-(4-methyl-3-pentenyl)-3-cyclohexenecarbaldehyde (52474-60-9); Prenyl benzoate (5205-11-8); Acetic acid, 2-methyl-6-methylene-7-octen-2-yl ester (1118-39-4); Pseudocumene (95-63-6); 2-Cyclopentylcyclopentyl crotonate (68039-73-6); Methyl-beta-ionone (127-43-5); Phenylacetaldehyde hexylene glycol acetal (67633-94-7); 1-Decanol (112-30-1); Rhodinyl phenylacetate (10486-14-3); 2,4-Dimethyl-4-phenyltetrahydrofuran (82461-14-1); 3',6(7)-Dimethyl-hexahydro-spiro-1,4-methanonapthalene-2(1H)-2'-oxirane (41816-03-9); acetic acid 2 -(1-oxopropoxy)- 1- (3,3 dimethyl cyclohexyl) ethyl ester (236391-76-7); 2-tert.Butyl-4-methyl-cyclohexanol (67634-11-1); Trichloromethyl phenyl carbinyl acetate (90-17-5); 2-Methyl-5-phenyl-1-pentanol (25634-93-9); 2-Methyl-4-(2,2,3-trimethylcyclopent-3-en-1-yl)-2-buten-1-ol (28219-60-5); 5-(2,2,3-Pentamethyl-3-cyclopentenyl)-3-methylpentan-2-ol (65113-99-7); 3-(2-camphenyl) iso cyclohexanol (70955-71-4); 3-Isocamphylcyclohexanol (mixture of isomers) (3407-42-9); 6(5)-Methoxy dicyclopentadiene carboxaldehyde (86803-90-9); propyl (2S)-2-(1,1-dimethylpropoxy) propanoate (319002-92-1); 5-Methyl-3-heptanone oxime (22457-23-4); 2-Ethylhexanal cycloglycolacetal (4359-47-1); Isolongifolanol (57566-26-4); 3,7-Dimethyloctanal (5988-91-0)Tetrahydrojasmone (13074-63-0); Tetrahydrolinalool (78-69-3); 3,7-Dimethyloctan-3-yl acetate (20780-48-7); 3,7-dimethyloct-1-en-2-ol (41678-36-8); Tetrahydromyrcenol (18479-57-7); ethyl (1R,6S)-2,2,6-trimethylcyclohexanecarboxylate (22471-55-2); Tridecanol (112-70-9); 2-Tridecenenitrile (22629-49-8); 2,4-Dimethyl-3-cyclohexene-1-carboxaldehyde (68039-49-6); 4-Methyl-3-decen-5-ol (81782-77-6); (4-formyl-2-methoxyphenyl) 2-methylpropanoate (20665-85-4); Propenylguaethol (94-86-0); 3,5,5-Trimethylhexyl acetate (58430-94-7); 2-Pentyl-2,5,5-trimethylcyclopentanone (65443-14-3); Cyclohexadec-5-enone (37609-25-9); Verbenyl acetate (33522-69-9); Hexahydro-4,7-methanodinden-5(6)-yl methyl ether (27135-90-6); Methyl N-2-methyl-3-(4-tert.butylphenyl)propylidenanthranilate (91-51-0); 2-tert.Butylcyclohexanol (13491-79-7); 4-Isopropyl-1-methyl-2-propenylbenzene (14374-92-6); 2-tert.Butylcyclohexyl acetate (88-41-5); 1-Formyl-1-methyl-4-(4-methylpentyl)-3-cyclohexene (66327-54-6); 7-Acetyl-6-ethyl-1,1,4,4-tetramethyl-1,2,3,4-tetrahydronaphthalene (88-29-9); 4-tert.Butylcyclohexyl acetate (32210-23-4); Methyl cedryl ketone (32388-55-9); N-2,4-Dimethyl-3-cyclohexenemethylene methyl anthranilate (68738-99-8); 4-Methyl-4-phenyl-2-pentanone (7403-42-1); Caryophyllene acetate (57082-24-3); beta-Naphthyl methyl ether (93-04-9); 2-Hexyl-1,3-dioxolane (1708-34-5); Methyl isobutyl carbinyl crotonate (35206-51-0) and mixtures thereof.

### Optional Ingredients

The compositions herein can contain a variety of other optional components suitable for rendering such compositions more cosmetically or aesthetically acceptable or to provide them with additional usage benefits. Such conventional optional ingredients are well-known to those skilled in the art. These include any cosmetically acceptable ingredients such as those found in the CTFA International Cosmetic Ingredient Dictionary and Handbook, 7th edition, edited by Wenninger and McEwen, (The Cosmetic, Toiletry, and Fragrance Association, Inc., Washington, D.C., 1997*).* As used herein "cosmetically acceptable" means a material (e.g., compound or composition) which is suitable for use in contact with skin, hair or other suitable substrates such as hard surfaces and fabrics as may be applicable to the product form and as defined hereinbelow.

### Cosmetically Acceptable Carrier

The fragrance compositions of the present invention will preferably comprise a cosmetically acceptable carrier. The phrase "cosmetically acceptable carrier", as used herein, means one or more compatible solid or liquid fillers, diluents, extenders and the like, which are cosmetically acceptable and compatible with the fragrance composition. The term "compatible", as used herein, means that the components of the compositions of this invention are capable of being combined with the additional actives , in a manner such that there is no interaction which would substantially reduce the efficacy of the composition under ordinary use situations. The type of carrier utilized in the present invention depends on the type of product desired. The compositions useful in the present invention may be a wide variety of product types. These include, but are not limited to, oils, lotions, creams, gels, sticks, sprays, ointments, pastes, mousses and cosmetics (e.g., solid, semi-solid, or liquid make-up, including foundations). These product types may comprise several types of carriers including, but not limited to, solutions, aerosols, emulsions (including oil-in-water or water-in-oil), gels, solids, capsules, microcapsules and liposomes.

In alternative applications such as household products such as laundry detergents, dishwashing and fabric softening products, alternative forms such as granules, liquid concentrates and pouches may be used.

### Volatile Solvent

The fragrance compositions of the present invention may also comprise one or more volatile solvent. If present, the volatile solvent or mixture of volatile solvents will generally be at a level of about 5% or greater, preferably about 10% or greater, more preferably about 20% or greater, even more preferably about 50% or greater, by weight of the total composition. The solvents useful herein are preferably organic volatile solvents.

As used herein, "volatile" refers to substances with a significant amount of vapour pressure under ambient conditions, as is understood by those in the art. The volatile solvents for use herein will preferably have a vapour pressure of about 2kPa or more, more preferably about 6kPa or more, at 25°C. The volatile solvents for use herein will preferably have a boiling point under one atmosphere of less than about 150°C, more preferably less than about 100°C, even more preferably less than about 90°C, even more preferably still less than about 80°C.

Preferably the volatile solvents for use herein will be relatively odourless and safe for use on human skin. Suitable volatile solvents include, but are not limited to, C₁-C₄ alcohols, volatile silicones and mixtures thereof. Preferred volatile solvents are C₁-C₄ alcohols and mixtures thereof. More preferred for use herein is ethanol. Typically, the compositions may comprise from 50 to 75% of volatile solvents.

### Non-volatile solvents

While the fragrance compositions of the present invention preferably comprise a volatile solvent they may also comprise "non-volatile" solvents. Suitable non-volatile solvents include, but are not limited to, benzyl benzoate, diethyl pthalate, isopropyl myristate, propylene glycol, dipropylene glycol and mixtures thereof.

### Water

The fragrance compositions of the present invention may also comprise water. If present, the water will preferably comprise from about 0.1 % to about 40%, more preferably from about 1% to about 30%, even more preferably about 5% to about 20%, by weight of the total composition.

### Additional Ingredients

There are a number of optional additional ingredients that are suitable for inclusion into the present fragrance compositions, which will be dependent upon the product type such as personal beauty care or household cleaners and laundry products for example and readily know to those skilled in the art. These include, but are not limited to, alcohol denaturants such as denatonium benzoate; UV stabilisers such as benzophenone-2; antioxidants such as tocopheryl acetate; radical scavengers; preservatives such as phenoxyethanol, benzyl alcohol, methyl paraben, propyl paraben; sunscreens; dyes; pH adjusting agents such as lactic acid, citric acid, sodium citrate, succinic acid, phosphoric acid, sodium hydroxide, sodium carbonate; deodorants and anti-microbials such as farnesol and zinc phenolsulphonate; antiperspirant actives include, for example, the aluminum and zirconium salts, such as aluminum halides, aluminum hydroxyhalides, zirconyl oxyhalides, zirconyl hydroxyhalides, and mixtures thereof, humectants such as glycerine; oils; moisturisers; anti inflammatory agents; skin conditioning agents such as allantoin; cooling agents such as trimethyl isopropyl butanamide and menthol; surfactants; polymers; dispersing agents; lubricants; hair conditioning ingredients such as panthenol, panthetine, pantotheine, panthenyl ethyl ether, and combinations thereof; silicones; solvents such as hexylene glycol; hair-hold polymers such as those described in WO-A-94/08557; salts in general, such as potassium acetate and sodium chloride and mixtures thereof. If present, these additional ingredients will preferably be present at a level of less than 10%, by weight of the total composition. More preferably these additional ingredients will be present at a level of less than 5%, by weight of the total composition.

### Product Forms

The fragrance compositions of the present invention may take any form suitable for the intended use. For cosmetic use these may include, but are not limited to, vapour sprays, aerosols, emulsions, solid sticks, lotions, oils and liquids. Preferably, the compositions of the present invention take the form of a vapour spray, aerosol dispenser or non-aerosol pump-spray dispenser. Most preferably the invention takes the form of a vapour spray.

### Autonomic Nervous System (ANS)

The method of the present invention enables the formulator of fragrance compositions to evaluate and profile a fragrance composition based upon the ANS response to the composition.

There are a number of Autonomic Nervous System (ANS) response parameters which may be measured in order to apply random/bootstrap forest statistical analysis including: electrodermal indices, such as skin conductance, and thermovascular indices such as skin temperature (ST), respiratory rate (RR), heart rate (HR), and blood pressure (BP diastolic & systolic). Preferably at least 4 ANS parameters are used, more preferably at least 5 ANS parameters selected from those listed above, preferably at least blood pressure and skin temperature.

Skin conductance, also known as galvanic skin response (GSR), electrodermal response (EDR), psychogalvanic reflex (PGR), skin conductance response (SCR) or skin conductance level (SCL), is a method of measuring the electrical conductance of the skin, which varies with its moisture level. The sweat glands are controlled by the sympathetic nervous system, and thus skin conductance may be used as an indication of psychological or physiological arousal.

Skin temperature is the response of the ANS to changes in psychological state and can be detected through measurement of signals reflecting dermal activity.

Respiratory rate (RR, Vf or Rf) is also known by respiration rate, pulmonary ventilation rate, ventilation rate, or breathing frequency and is the number of breaths taken within a set amount of time, typically 60 seconds.

Heart rate (HR) is the number of heart beats per unit time, usually expressed as "beats per minute" (b.p.m.).

Blood pressure (BP diastolic & systolic) refers to the arterial pressure of the systemic circulation. During each heartbeat, blood pressure varies between a maximum (systolic) and a minimum (diastolic) pressure. The blood pressure in the circulation is principally due to the pumping action of the heart. The measurement blood pressure refers to the systemic arterial pressure measured at the upper arm and is a measure of the pressure in the brachial artery, a major artery in the upper arm. Blood pressure is usually expressed in terms of the systolic pressure over diastolic pressure and is measured in millimeters of mercury (mmHg), for example 140/90.

### Testing Equipment

The ANS response parameters may be measured as described below.
(1) Respiration rate is measured using a piezo-electric transducer (UFI, Morro Bay, Ca, US). The transducer works by measuring the changes to chest circumference during breathing. The information gathered from the transducer is sent to a pre-amplifier (CED1902, Cambridge Electronic Design, Cambridge, UK), the data were then digitized (CED1401 laboratory interface, Cambridge Electronic Design, Cambridge, UK) and analyzed on a computer using the data handling program Spike 2 (Version 7.0, Cambridge Electronic Design, Cambridge, UK).
(2) Heart rate is measured by a pulse oximeter (SIMS Graseby, Watford, Herts, UK). The pulse oximeter produces a photoplethysmograph and output from this device was analyzed with Spike 2 (Cambridge Electronic Design, Cambridge, UK). The pulse oximeter probe was placed on the index finger of the participant's right hand.
(3) Skin resistance is recorded using a conductance meter (CED2502SA , Cambridge Electronic Design, Cambridge, UK). This conductance meter will measure the conductivity between the electrodes and the skin of a participant. The electrodes were placed on the third and fourth finger of the participant's right hand. The more ionic sweat that is produced from a participant the more conductive the skin becomes. Therefore skin conductance is approximate to the amount of sweat produced by a participant. Skin conductance (1/resistance) output is generated and analysed by Spike 2 (Cambridge Electronic Design, Cambridge, UK).
(4) Blood pressure is measured using a sphygmomanometer or blood pressure meter (also referred to as a sphygmometer). This is a device used to measure diastolic and systolic blood pressure, composed of an inflatable cuff to restrict blood flow, and a mechanical manometer to measure the pressure. Using an upper arm cuff monitor (OMRON M10-IT, Boots, UK), placed on the right arm of the participant systolic/diastolic blood pressure was taken as described in the protocol.
(5) Skin temperature is measured using a thermistor (RS Components Ltd. Corby, UK). The output of the thermistor thermistor was sent to a pre-amplifier (CED1902, Cambridge Electronic Design, Cambridge, UK), the data were then transduced into a digital signal (CED1401 laboratory interface, Cambridge Electronic Design, Cambridge, UK) and analyzed on a computer using the data handling program Spike 2 (Cambridge Electronic Design, Cambridge, UK).

### ANS Response Testing

The method to evaluate and classify a fragrance composition according to the associated biometric response in humans comprises the steps of selecting at least 20 test participants (N), a control and at least 2 fragrance compositions. Preferably in a controlled environment, each test participant is exposed to the control and each predetermined fragrance composition for a predetermined time period in a randomized order. During this period at least 4, or preferably 5, biometric ANS parameters selected from the group above are measured for the test participant before and after the exposure to each of said predetermined fragrance compositions. An associated biometric fingerprint of each of said predetermined fragrance compositions from said biometric ANS parameters of all participants is subsequently defined. Partition and random/bootstrap forest statistical analysis methods are then applied to the associated biometric fingerprint data to create a classification model based upon the data, whereby a predetermined fragrance composition tested by a test participant based upon ANS data can be identified. It is believed that the use of at least 20 participants and 4 to 5 ANS parameters results in the preferable number of data inputs (i.e. 80 to 100) for the statistical analysis to produce a reliable model. This method enables a correct prediction of at least 75% with 4 ANS measures, more preferably at least 79% with 5 ANS measures, even more preferably at least 95% with all 6 ANS measures.

### ANS testing protocol

### 20 to 23 Female test participants

### Age 18-28 years old

1. Test participant arrives at the test centre laboratory.
2. Lab conditions: humidity = 35-40%, temperature 22±1°C.
3. Test participant is seated and given the Information Sheet explaining the experiment.
4. Participant signs a Consent Form and completes a Medical Questionnaire.
5. Set up biometric equipment and connect to test participant. They are given 5 minutes to relax.
6. Participant blind folded.
7. Start the biometric equipment.
8. Label scent strip and dip (4-5mm) into the randomized fragrance composition (or control) and set the timer for 5mins.
9. Record baseline (no stimulus) - 2 mins.
10. Put the scent strip (after 5 mins drying) in the holder in front of the subject at circa 2.5 cm from nose.
11. Record all biometric data for 2 mins - first condition.
12. Remove scent strip and blindfold.
13. Rest period of 5 mins.
14. Repeat 6-13 steps with subsequent conditions.

Whilst not bound by theory, it is preferred that test participants should not be exposed to more than 2 test fragrance composition in any test session. The test participants may participate in 2 or more test sessions per day with an hour break between them and or over a number of days, which may or may not necessarily be consecutive. Test participants should preferably not test more than 4 fragrances per day. Presentation of the 2 fragrances and control in any test should also be randomized.

Where the fragrance compositions are "Eau de Toilette" compositions, a blotter can be dipped into the composition and allowed to dry for 5mins prior to being administered to a test participant. For non fragrance compositions such as shampoos or detergents if the composition does not allow drying on a blotter, about 2-5g of the composition should be placed in an open container for 5mins prior to exposure to the test participant.

### Fragrance compositions tested:

The following fragrance compositions commercially available in the European Union, between 2010 and 2013, of a variety of characters containing at least 5 PRMS and considered as pleasant fragrances (as defined hereinafter) were used for ANS response testing and data generation:

| No. | Fragrance | Brand | No. | Fragrance | Brand |
|---|---|---|---|---|---|
| 1. | Miss Dior Cherie | Christian Dior | 6. | Valentina | Valentino |
| 2. | Coco Mademoiselle | Chanel | 7. | Daisy | Marc Jacobs |
| 3. | Acqua di Gioia | Giorgio Armani | 8. | Hugo Woman | Hugo Boss |
| 4. | Guilty | Gucci | 9. | Bombshell | Victoria's Secret |
| 5. | Light Blue | Dolce & Gabbana | 10. | Eau de Rochas | Rochas |

Control composition: 75% ethanol and 25% water

The test fragrances were also assessed by a consumer test panel for 'Overall, how would you rate the fragrance of this Eau de Toilette for your own use' for use on skin. The consumer test panelists were provided with an unlabelled/branded fragrance bottle which they were asked to apply to the skin in their usual manner in the morning and at the end of the day rate the fragrance using the scale as described in Table 1.

**Table 1**

| 1 | 2 | 3 | 4 | 5 |
|---|---|---|---|---|
| Poor | Fair | Good | Very Good | Excellent |

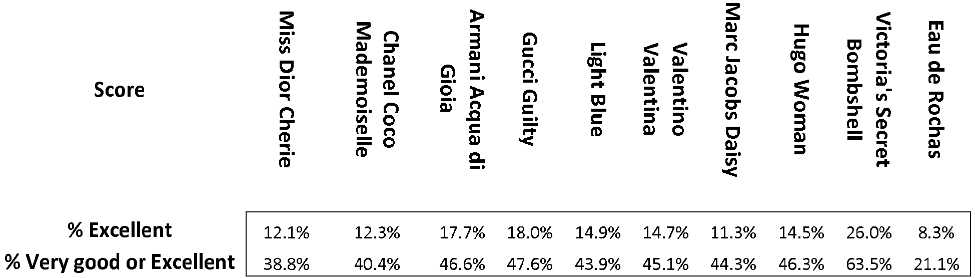

It has been surprisingly found that the use of partition methods (in particular random/bootstrap forest methods) of statistical analysis can effectively classify the ANS data. Simple partition methods build decision trees. This method is expanded further using random/bootstrap forest methods. The random/bootstrap forest method makes many trees (randomly selected with replacement from the dataset), and combines the models to obtain the final predictive model. This method is particularly advantageous as the data are analyzed without the necessity to make certain statistical assumptions that are required for other classification techniques. A number of statistical package programs are available to conduct this analysis.

Statistical discovery software JMP PRO version 11 available from SAS was used to model the data using the partition methods platform. Default settings were utilized. However, other packages suitable for use include SAS (TREEDISC macro or enterprise miner), R (rPart or rRandom Forest commands) and Excel add in (XLStat).

### Results

The analysis of the data from the 10 fragrances and all ANS parameters are shown below. The results of the applying the partition method analysis verses the actual composition tested are shown below whereby the number of participants testing a given fragrance correctly identified by the model can be seen. It clearly shows that the model correctly predicts 95% using all 6 ANS parameters (skin conductance, systolic and diastolic blood pressure, skin temperature, respiratory rate and heart rate).
Table: Number of participants predicted by the 6 ANS parameter model to have tested each fragrance.

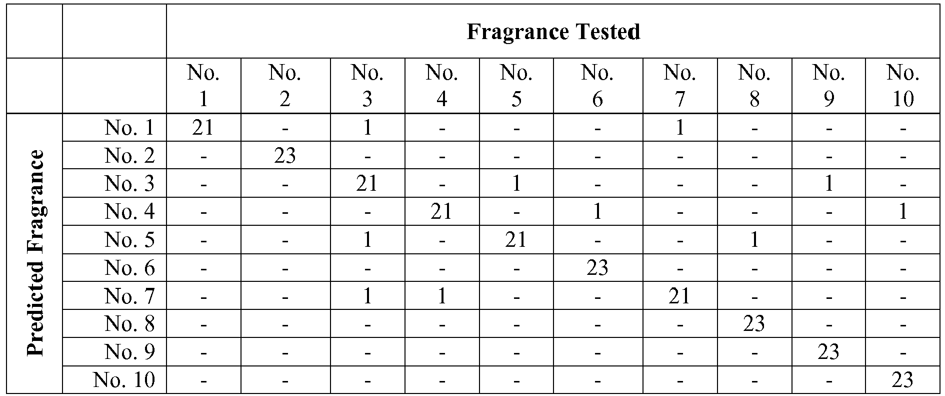

Good predictive results are also obtained by looking at 5 of the 6 ANS measures. Predictability ranges from 79% to 90% depending on the fragrance tested.

The same can be said when taking 4 of the ANS measures with predictability between 76% and 86% depending on the fragrance tested. As an example the predictions for the 10 fragrances when looking at 4 of the ANS measures (skin conductance, systolic blood pressure, skin temperature and heart rate) which correctly classifies 77% is given below.
Table: Number of participants predicted by the 4 ANS parameter model to have tested each fragrance.

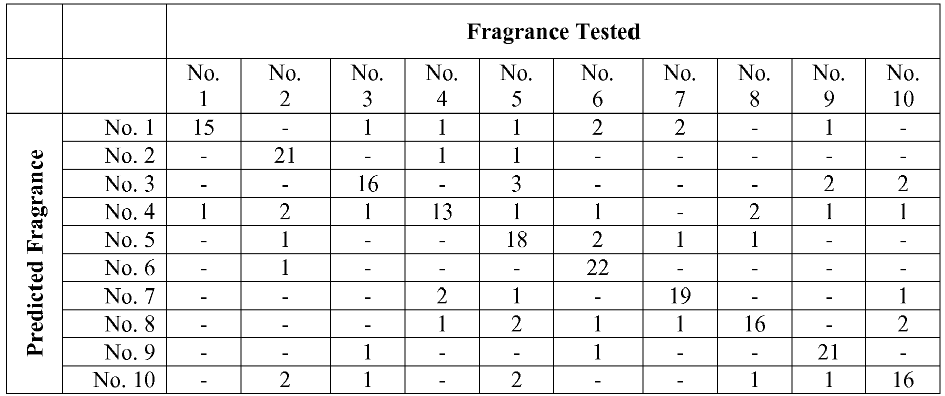

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method to evaluate and profile a fragrance composition according to the associated biometric response in humans to said composition comprising the steps of:
i) Selecting at least 20 test participants, a control composition and at least 2 fragrance compositions;
ii) Exposing each test participant to at least 3 conditions including a control and 2 predetermined fragrance compositions for a pre-determined time period;
iii) Measuring at least 4 biometric Autonomic Nervous System (ANS) parameters before and after the exposure to each predetermined fragrance composition to define an associated biometric fingerprint; and
iv) Applying partition and random/bootstrap forest statistical analysis methods to said associated biometric fingerprint data to create a profiling model based upon the data, whereby a predetermined fragrance composition tested by a test participant based upon ANS data can be identified.

2. The method according to claim 1, wherein said ANS parameters are selected from skin conductance, skin temperature, respiratory rate, heart rate, and systolic and diastolic blood pressure.

3. The method according to claim 2, wherein at least 5 of said ANS parameters are measured.

4. The method according to claim 3, wherein at least 6 of said ANS parameters are measured.

5. The method according to claim 1, wherein at least 4 fragrance compositions are selected.

6. The method according to claim 5, wherein each of said fragrances comprises at least 5 fragrance raw materials.

7. The method according to claim 1, wherein each of said fragrance compositions comprises a C1 to C5 alcohol.

8. The method according to claim 1, wherein each of said fragrance compositions comprises at least one synthetic perfume raw material.

9. The method according to claim 1, wherein each of said fragrance compositions is substantially free of naturally derived fragrance materials.

10. The method according to claim 1, wherein at least 70% of tested fragrances are correctly identified.

11. The method according to claim 10, wherein at least 75% of tested fragrances are correctly identified.
